(19) Europäisches Patentamt
European Patent Office
Office européen des brevets

(11) Veröffentlichungsnummer: **0 039 859**
**B1**

(12) # EUROPÄISCHE PATENTSCHRIFT

(45) Veröffentlichungstag der Patentschrift: **16.07.86**

(51) Int. Cl.[4]: **C 08 G 65/32**, C 08 G 4/00, C 11 D 1/722 // C11D1/76

(21) Anmeldenummer: **81103329.9**

(22) Anmeldetag: **02.05.81**

(54) **Verfahren zur Herstellung von Polyglykolethermischformalen und neue Polyglykolethermischformale.**

(30) Priorität: **12.05.80 DE 3018135**

(43) Veröffentlichungstag der Anmeldung: **18.11.81 Patentblatt 81/46**

(45) Bekanntmachung des Hinweises auf die Patenterteilung: **16.07.86 Patentblatt 86/29**

(84) Benannte Vertragsstaaten: **AT BE CH DE FR GB IT LI NL SE**

(56) Entgegenhaltungen:
**DE - A - 2 523 588**
**DE - A - 2 748 126**
**DE - C - 1 225 800**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Schmid, Karl, Dr., Stifterstrasse 10, D-4020 Mettmann (DE)**
Erfinder: **Grünert, geb. Hartlieb, Margarete, Gleiwitzer Strasse 4, D-4044 Kaarst 2 (DE)**
Erfinder: **Heidrich, Jochen, Dr., Robert-Koch-Strasse 41, D-4019 Monheim (DE)**
Erfinder: **Tesmann, Holger, Dr., Vennstrasse 61, D-4000 Düsseldorf 12 (DE)**

**EP 0 039 859 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung ist ein verbessertes Verfahren zur Herstellung von Polyglykolethermischformalen durch Umsetzung von Polyglykolethern mit symmetrischen, aus Formaldehyd und Alkoholen, Alkylglykolethern und Alkylpolglykolethern gebildeten Acetalen.

Aus der DE-OS 2 523 588 ist ein Verfahren zur Herstellung von Polyglykolethermischformalen bekannt, bei dem man Anlagerungsprodukte von Alkylenoxiden an langkettige aliphatische Alkohole oder Mono-, Di- und Trialkylphenole mit einem kurzkettigen Alkohol und Formaldehyd umsetzt. Die hierbei erhaltenen Produkte besitzen oberflächenaktive Eigenschaften und eignen sich als Tenside für Netz-, Wasch- und Reinigungsmittel. Als endgruppenverschlossene Polyglykoletherderivate bieten diese Substanzen im Vergleich zu Polyglykolethern mit freien Hydroxylgruppen entscheidende Vorteile wie Schaumarmut und hohe Alkalistabilität bei gleichzeitig vorhandener biologischer Abbaubarkeit.

Bei der Herstellung der Polyglykolether nach dem Verfahren der DE-OS 2 523 588 wird 1 Mol Alkyl-, Alkenyl- oder Phenylpolyglykolether mit 3 bis 5 Mol eines Alkohols und 0,5 bis 1 Mol Formaldehyd in Gegenwart einer starken Säure in der Hitze unter Auskreisen des gebildeten Dialkylformals und des Reaktionswassers umgesetzt. Es hat sich gezeigt, dass die auf diese Weise hergestellten Polyglykolethermischformale mit erheblichen Mengen Polyglykolether verunreinigt sind. Aus diesem Grund besitzen die so erhaltenen Produkte nur eine geringe Alkalistabilität und verfärben sich beim Lagern über gepulvertem Ätznatron bei 90 °C im Verlauf von 24 Stunden nach braun bis schwarz. Ein weiterer Nachteil dieser Produkte ist ihr Geruch nach Formaldehyd.

Der vorliegenden Erfindung lag die Aufgabe zugrunde ein verbessertes Verfahren zur Herstellung von Polyglykolethermischformalen zu finden, das hochreine, alkalistabile und geruchlose Produkte hoher Farbqualität liefert. Diese Aufgabe wird durch das nachstehend beschriebene Verfahren gelöst.

Gegenstand der Erfindung ist ein Verfahren zur Herstellung von Polyglykolethermischformalen der Formel I oder II,

$$\begin{array}{l} R^1\text{–CH–O–(A–O)}_m\text{–CH}_2\text{–(O–B)}_q\text{–O–R}^4 \\ \quad | \\ R^2\text{–CH–O–(A–O)}_n\text{–CH}_2\text{–(O–B)}_q\text{–O–R}^4 \end{array} \qquad I$$

$$R^3\text{–O–(A–O)}_p\text{–CH}_2\text{–(O–B)}_q\text{–O–R}^4 \qquad II$$

in denen $R^1$ für einen geradkettigen oder verzweigten Alkylrest mit 1 bis 18 Kohlenstoffatomen und $R^2$ für Wasserstoff oder einen geradkettigen oder verzweigten Alkylrest mit 1 bis 17 Kohlenstoffatomen steht, wobei die Summe der Kohlenstoffatome in $R^1$ und $R^2$ 6 bis 18 beträgt, $R^3$ einen Mono-, Di- oder Trialkylphenylrest mit 14 bis 26 Kohlenstoffatomen, einen geradkettigen oder verzweigten Alkylrest mit 8 bis 22 Kohlenstoffatomen oder einen Rest der Formel III bedeutet,

$$\begin{array}{l} R^1\text{–CH–O–R}^5 \\ \quad | \\ R^2\text{–CH–} \end{array} \qquad III$$

in der $R^1$ und $R^2$ die oben angegebene Bedeutung haben und $R^5$ einen geradkettigen oder verzweigten Alkylrest mit 1 bis 5 Kohlenstoffatomen, einen Aryl- oder Alkylarylrest mit 6 bis 9 Kohlenstoffatomen darstellt, während $R^4$ einen geradkettigen oder verzweigten Alkylrest mit 1 bis 5 Kohlenstoffatomen, A und B Ethylen- oder Isopropylenreste, m und n Zahlen von 0 bis 50, p eine Zahl von 2 bis 50 und q eine Zahl von 0 bis 3 bedeuten, wobei die Summe von $m + n$ 2 bis 50 beträgt, dadurch gekennzeichnet, dass man Polyglykolether der Formel IV oder V,

$$\begin{array}{l} R^1\text{–CH–O–(A–O)}_m\text{–H} \\ \quad | \\ R^2\text{–CH–O–(A–O)}_n\text{–H} \end{array} \qquad IV$$

$$R^3\text{–O–(/ZO)}_p\text{–H} \qquad V$$

in denen $R^1$, $R^2$, $R^3$, A, m, n und p die oben angegebene Bedeutung haben, mit einem Diformal der Formel VI,

$$R^4\text{–O–(B–O)}_q\text{–CH}_2\text{–(O–B)}_q\text{–O–R}^4 \qquad VI$$

in der $R^4$, B und q die oben angegebene Bedeutung haben, bei 60 bis 150 °C in Gegenwart einer starken Säure umsetzt, wobei pro Mol in IV oder V vorhandener Hydroxylgruppen 2 bis 10 Mol Diformal eingesetzt werden, den bei der Reaktion entstandenen Alkohol $R^4$–O–(B–O)$_q$H, gegebenenfalls zusammen mit Diformal, aus dem Reaktionsgemisch abdestilliert, die im Rückstand vorhandene Säure neutralisiert oder gegebenenfalls durch Filtration entfernt, das noch vorhandene Diformal abdestilliert und gegebenenfalls die anwesenden Salze durch Filtration entfernt.

Die als Ausgangsmaterial verwendeten Polyglykolether der Formel IV und V sind an sich bekannte Substanzen, die nach bekannten Methoden durch Anlagerung von Ethylenoxid und Propylenoxid an vicinale Alkandiole, Monoalkyl- oder Monoarylether von vicinalen Alkandiolen, Mono-, Di- oder Trialkylphenole und langkettige aliphatische Alkohole erhalten werden können.

Vicinale Alkandiole können unter anderem durch Epoxydation entsprechender Olefine und nachfolgende Hydrolyse der resultierenden Epoxyalkane erhalten werden. Als Beispiele für Alkandiole, die sich für die Herstellung der oben erwähnten Polyglykolether eignen seien hier genannt: Octandiol-1,2, Nonandiol-1,2, Decandiol-1,2, Dodecandiol-1,2, Hexadecandiol-1,2, Octadecandiol-1,2, Eikosandiol-1,2, Gemische aus Alkandiolen-1,2 der Kettenlänge $C_{12}$–$C_{14}$, Gemische aus Alkandiolen-1,2 der Kettenlänge $C_{16}$–$C_{18}$, Gemische aus isomeren vicinalen Alkandiolen der Kettenlänge $C_{10}$ mit nichtendständigen Hydroxylgruppen, Gemische aus isomeren vicinalen Alkandiolen der Kettenlänge $C_{18}$ mit nichtendständigen Hydroxylgruppen, Gemische aus isomeren vicinalen Alkandiolen der Kettenlänge $C_{11}$–$C_{15}$ mit nichtendständigen Hydroxylgruppen, Gemische aus isomeren vicinalen Alkandiolen der Kettenlänge $C_{14}$–$C_{16}$ mit nichtendständigen Hydroxylgruppen, und Gemische aus isomeren vicinalen Alkandiolen der Kettenlänge $C_{15}$–$C_{18}$ mit nichtendständigen Hydroxylgruppen.

Monoalkyl-, Monoaryl- und Monoalkylarylether der oben erwähnten Alkandiole können beispielsweise aus entsprechenden Epoxyalkanen durch Umsetzung mit aliphatischen Alkoholen, Phenol und Alkylphenolen erhalten werden. Dabei kommen als Epoxyalkane unter anderem folgende Verbindungen in Betracht: 1,2-Epoxyoctan, 1,2-Epoxydodecan, 1,2-Epoxytetradecan, 1,2-Epoxyhexadecan, 1,2-Epoxyoctadecan, 1,2-Epoxyeikosan, Gemische aus 1,2-Epoxyalkanen der Kettenlänge $C_{12}$–$C_{14}$, Gemische aus 1,2-Epoxyalkanen der Kettenlänge $C_{16}$–$C_{18}$, Gemische aus isomeren Epoxyalkanen der Kettenlänge $C_{10}$ mit nichtendständigen Epoxygruppen, Gemische aus isomeren Epoxyalkanen der Kettenlänge $C_{18}$ mit nichtendständigen Epoxygruppen, Gemische aus isomeren Epoxyalkanen der Kettenlänge $C_{11}$–$C_{15}$ mit nichtendständigen Epoxygruppen, Gemische aus isomeren Epoxyalkanen der Kettenlänge $C_{14}$–$C_{16}$ mit nichtendständigen Hydroxylgruppen und Gemische aus isomeren Epoxyalkanen der Kettenlänge $C_{15}$–$C_{18}$ mit nichtendständigen Epoxygruppen. Für die Umsetzung dieser Epoxyalkane zu den entsprechenden beta-Hydroxyethern eignen sich beispielsweise Methanol, Ethanol, Propanol, Butanol, Phenol, o-Kresol, p-Kresol, Ethylenglykol und Isopropylenglykol.

Als Phenole, die sich für die Herstellung der oben erwähnten Polyglykolether eignen, kommen unter anderen in Betracht: Octylphenol, Octylkresol, Nonylphenol, Dodecylphenol, Tributylphenol und Dinonylphenol.

Beispiele für geeignete Fettalkohole sind n-Octanol, n-Decanol, n-Dodecanol, n-Tetradecanol, n-Hexadecanol und n-Octadecanol. In der Regel verwendet man zu Synthese der als Ausgangsmaterial eingesetzten Polyglykolether jedoch Fettalkoholgemische, wie sie bei der Natriumreduktion oder der katalytischen Hydrierung von Fettsäuregemischen nativer Fette, Öle und Wachse erhalten werden, beispielsweise die technischen Kokos-, Palmkern-, Talg-, Sojaöl- und Leinölfettalkohole. Weiterhin kommen Gemische synthetischer Alkohole in Betracht, die aus Erdölprodukten nach dem Ziegler- oder dem Oxo-Verfahren hergestellt werden, ferner Gemische vorwiegend sekundärer Alkohole, die durch Luftoxydation geradkettigen Paraffine in Gegenwart von Borsäure oder Borsäureanhydrid hergestellt werden.

Zur Herstellung der als Ausgangsmaterial verwendeten Polyglykolether werden die hydroxylgruppenhaltigen Verbindungen in bekannter Weise bei erhöhter Temperatur und erhöhtem Druck in Gegenwart von geeigneten Alkoxylierungskatalysatoren mit entsprechenden Mengen Alkylenoxid umgesetzt. Die Epoxide können einzeln oder miteinander gemischt zur Reaktion gebracht werden. Sie können auch nacheinander in beliebiger Reihenfolge zum Einsatz kommen. Unter den gemischten Ethylenoxid-/Propylenoxidanlagerungsprodukten werden, wegen ihrer günstigen anwendungstechnischen Eigenschaften, solche bevorzugt, in denen die Alkoxyeinheiten bis zu 20 Mol-% aus Isopropoxyeinheiten bestehen.

Die bei der Durchführung des erfindungsgemässen Verfahrens weiterhin als Ausgangsmaterial verwendeten Diformale der Formel VI stellen ebenfalls eine bekannte Substanzklasse dar. Ihre Herstellung erfolgt zweckmässigerweise durch Acetalisieren von Formaldehyd mit Alkoholen oder Alkylalkylenglykolethern bei erhöhter Temperatur und in Gegenwart einer starken Säure. Als Alkohole kommen vor allem Methanol, Ethanol, n-Propanol und insbesondere Butanol in Betracht. Beispiel für geeignete Alkylalkylenglykolether sind die Anlagerungsprodukte von 1 bis 3 Mol Ethylenoxid oder Propylenoxid an die vorgenannten Alkohole.

Bei der Durchführung des erfindungsgemässen Verfahrens lässt man die Reaktionspartner in Gegenwart einer starken Säure aufeinander einwirken. Hier werden mit Vorteil Phosphorsäure, p-Toluolsulfonsäure, saure Ionenaustauscher und Zeolithe und vorzugsweise Schwefelsäure verwendet. Es hat sich dabei als zweckmässig erwiesen, Säuremengen im Bereich von 0,05 bis 0,5 Gewichtsprozent bezogen auf umzusetzenden Polyglykolether, einzusetzen.

Das erfindungsgemässe Verfahren wird in Reaktionsgefässen durchgeführt, die ein Abdestillieren des entstehenden freien Alkohols und des überschüssigen Diformals, vorzugsweise unter vermindertem Druck, gestatten. Bei Ansätzen im technischen Massstab ist es vorteilhaft, die Reaktionsmischung während des Erhitzens zu rühren. Die Reaktion wird im allgemeinen bei Temperaturen von 60 bis 150 °C, vorzugsweise bei 70 bis 100 °C, durchgeführt.

Ein wesentliches Merkmal des erfindungsgemässen Verfahrens besteht darin, dass man den bei der Reaktion zwischen dem Polyglykolether und dem symmetrischen Diformal entstehenden Alkohol vor der Neutralisation oder dem Abfiltrieren des sauren Katalysators aus dem Reaktionsgemisch entfernt. Dies kann in der Weise geschehen, dass man die Reaktionsmischung so lange erhitzt, bis die Reaktion zwischen Polyglykolether und symmetrischem Diformal beendet ist, und dann den entstandenen Alkohol abdestilliert. In einer Variante des Verfahrens wird der entstehende Alkohol aus dem reagierenden Gemisch laufend abgezogen. Bei der Verwendung von einfachen Destillationsvorrichtungen geht in der Regel ein Teil des im Überschuss vorhandenen Diformals mit dem freien Alkohol über. Eine saubere Abtrennung des gebildeten Alkohols kann mittels einer entsprechend dimensionierten Destillationskolonne bewirkt werden. Besonders günstig in dieser Hinsicht gestaltet sich das erfindungsgemässe Verfahren, wenn symmetrische Diformale der Formel VI eingesetzt werden, in denen q die Werte 1 bis 3 hat. Die in diesem Fall entstehenden Alkohole $R^4$–O–(B–O)$_q$–H können aus dem Reaktionsgemisch abgezogen werden, ohne dass Diformal mit überdestilliert.

Nach dem Entfernen des freien Alkohols wird die im Reaktionsgemisch vorhandene Säure durch Zugabe von basischen Stoffen wie Natrium- oder Kaliumhydroxid, Kaliumcarbonat oder vorzugsweise Natriummethylat in methanolischer Lösung neutralisiert. Im Anschluss daran wird das bei der Umsetzung nicht verbrauchte Diformal abdestilliert, vorzugsweise unter vermindertem Druck.

Die als Rückstand verbleibenden Mischformale können in den Fällen, in denen die vorhandenen Neutralsalze nicht stören, direkt der Verwendung zugeführt werden. Sofern die Abtrennung der Salze geboten erscheint, kann diese durch einfache Filtration, gegebenenfalls unter Verwendung eines Filtrierhilfsmittels bewirkt werden.

Die Polyalkylenglykolethermischformale der Formel I sowie die Verbindungen der Formel II, in der $R^3$ die in der Formel III wiedergegebene Bedeutung hat, sind neue Substanzen.

Die nach dem erfindungsgemässen Verfahren hergestellten Polyalkylenglykolethermischformale sind geruchlos und fallen mit OH-Zahlen im Bereich von 0 bis 6 an. Sie sind im Gegensatz zu Mischformalen, die nach einem herkömmlichen Verfahren hergestellt wurden, in Gegenwart von starken Alkalien wie Alkalihydroxiden, Alkalisilikaten und Alkaliphosphaten äusserst stabil und lagerbeständig. So können Proben der erfindungsgemäss hergestellten Mischformale 3 Tage lang bei 90 °C über Ätznatron aufbewahrt werden, ohne dass Zersetzung oder Verfärbung eintritt.

Die Mischformale der Formeln I und II stellen oberflächenaktive Substanzen mit ausgezeichneter Reinigungswirkung und äusserst geringer Schaumneigung dar. Darüberhinaus sind sie biologisch abbaubar. Aufgrund dieser Eigenschaften eignen sie sich insbesondere zur Herstellung von Spül- und Industriereinigungsmitteln. Weiterhin eignen sich die erfindungsgemäss hergestellten Mischformale zur Verwendung in flüssigen oder festen Wasch- und Reinigungsmitteln. Dabei können sie entweder allein oder in Kombination mit anderen bekannten nichtionischen, kationischen, anionischen oder zwitterionischen oberflächenaktiven Substanzen, Gerüstsubstanzen und anderen Zusatz- oder Hilfsstoffen in den Wasch-und Reinigungsmittelzusammensetzungen zur Verwendung kommen.

Der Gehalt der Wasch- und Reinigungsmittelformulierungen an Verbindungen der Formel I und II kann in weiten Grenzen schwanken. Je nach dem Einsatzzweck und den Einsatzbedingungen der Wasch- und Reinigungsmittel kann ihr Gehalt an Mischformalen zwischen 1 und 20 Gewichtsprozent, vorzugsweise 2 bis 10 Gewichtsprozent betragen.

Die nachfolgenden Beispiele sollen den Gegenstand der Erfindung erläutern, ihn jedoch nicht darauf beschränken.

Beispiel 1

In einem Rührkessel mit Rührer, Innenthermometer, Wasserscheider und Rückflusskühler wurden 86,2 kg (1162,7 Mol) Butanol, 19,6 kg (654,0 Mol) Paraformaldehyd und 54,5 ml konz. Schwefelsäure solange auf ca. 100 °C erhitzt, bis sich durch azeotrope Destillation 10,46 kg Wasser abgeschieden hatten. Nach dem Abkühlen wurde die vorhandene Säure durch Zugabe von 180 g 30gewichtsprozentiger Natriummethylatlösung neutralisiert. Nach Zugabe von 100 g festem Kaliumcarbonat wurde das Reaktionsprodukt fraktioniert destilliert. Bei 87 °C/15 mbar gingen 71 kg Dibutylformal (76% d. Th.) über.

55,8 kg (348,8 Mol) des erhaltenen Dibutylformals, 36,2 kg (58,1 Mol) eines Adduktes von 9 Molk Ethylenoxid an 1 Mol Kokosfettalkohol (Gemisch aus Fettalkoholen der Kettenlänge $C_{12}$–$C_{18}$) und 73 g konz. Schwefelsäure wurden in einer Destillationsapparatur 2 Stunden lang auf 80 °C erhitzt. Danach wurde in der Destillationsapparatur der Druck soweit vermindert, dass Butanol im Gemisch mit Dibutylformal abzudestillieren begann. Nach 2 Stunden waren 27,9 kg des Butanol-/Dibutylformalgemisches übergegangen. Das verbliebene Gemisch wurde mit Natriummethylat neutralisiert. Anschliessend wurde überschüssiges Dibutylformal abdestilliert, wobei die Sumpftemperatur bis auf 150 °C gesteigert wurde. Durch Filtration des Destillationsrückstandes wurden schliesslich 40 kg Polyglykolethermischformal mit einer OH-Zahl von 1,1 und einem Trübungspunkt von 24 °C erhalten.

Beispiel 2–12

Die Umsetzungen wurden in Analogie zu Beispiel 1 durchgeführt. Neben n-Dodecanolpolyethylenglykolethern wurden Ethylenoxid- und Ethylenoxid-/Propylenoxidanlagerungsprodukte von Fettalkoholgemischen als Ausgangsmaterial verwendet. Diese Polyalkylenglykolether sind in der nachfolgenden Tabelle I durch die Fettalkoholkomponente und die Anzahl der angelagerten Alkylenoxideinheiten (EO = Ethylenoxid; PO = Propylenoxid) charakterisiert. Bei den Ethylenoxid-/Propylenoxidanlagerungsprodukten ist die Reihenfolge der Anlagerung aus der Angabe ersichtlich; + 8 EO + 1 PO bedeutet, dass zuerst 8 Mol Ethylenoxid, dann 1 Mol Propylenoxid addiert wurden. In der vorletzten und letzten Spalte der Tabelle I sind die OH-Zahlen und die Trübungspunkte der erhaltenen Mischformale verzeichnet.

Beispiel 13

Als Ausgangsmaterial diente ein Polyalkylenglykolethergemisch, das durch Reaktion von Ethylenglykol mit einem 1,2-Epoxyalkangemisch der Kettenlänge $C_{12}$–$C_{18}$, Anlagerung von 1 Mol Propylenoxid und anschliessende Addition von 19 Mol Ethylenoxid erhalten worden war. Dieses Polyalkylenglykolethergemisch wurde wie in Beispiel 1 beschrieben mit Dibutylformal umgesetzt. Es wurde ein Mischformal mit der OH-Zahl 1,4 erhalten.

Beispiel 14

In einem Dreihalskolben mit Rührer, Innenthermometer, Wasserabscheider und Rückflusskühler wurden 4248 g (36 Mol) Butylglykol, 600 g (10 Mol)

Tabelle 1

| Beispiel | Polyglykolether | Dialkylformal | OH–Zahl | Trübungspunkt °C |
|---|---|---|---|---|
| 2 | Dodecanol + 5 EO | $(C_4H_9O)_2\ CH_2$ | 0,6 | < 0 |
| 3 | Dodecanol + 6 EO | $(C_4H_9O)_2\ CH_2$ | 1,8 | < 0 |
| 4 | Dodecanol + 10 EO | $(C_4H_9O)_2\ CH_2$ | 2,0 | 20 |
| 5 | $C_{12-16}$–Fettalkohol + 9 EO | $(C_4H_9O)_2\ CH_2$ | 0,3 | 19 |
| 6 | $C_{12-18}$–Fettalkohol + 7 EO | $(C_4H_9O)_2\ CH_2$ | 1,3 | 12 |
| 7 | $C_{12-18}$–Fettalkohol + 11 EO | $(C_4H_9O)_2\ CH_2$ | < 1 | 26 |
| 8 | $C_{12-18}$–Fettalkohol + 8 EO + 1 PO | $(C_4H_9O)_2\ CH_2$ | 1,6 | 17 |
| 9 | $C_{12-18}$–Fettalkohol + 1 PO + 8 EO | $(C_4H_9O)_2\ CH_2$ | < 1 | < 0 |
| 10 | $C_{16-18}$–Fettalkohol + 20 EO | $(C_4H_9O)_2\ CH_2$ | 1,1 | 50 |
| 11 | $C_{16-18}$–Fettalkohol + 30 EO | $(C_4H_9O)_2\ CH_2$ | < 1 | 62 |
| 12 | $C_{12-18}$–Fettalkohol + 9 EO | $(C_3H_7O)_2\ CH_2$ | 1,2 | 40 |

Paraformaldehyd und 9,2 g konz. Schwefelsäure in 500 ml Toluol solange auf 110 °C erhitzt, bis sich durch azeotrope Destillation 324 ml Wasser abgeschieden hatten. Nach dem Abkühlen wurde das Reaktionsgemisch wurdh Zugabe von Natriummethylatlösung auf pH 7 bis 8 neutralisiert. Bei der anschliessenden Destillation ging zunächst im Wasserstrahlvakuum ein Gemisch aus Toluol und Butylglykol über. Als Hauptlauf wurden 3400 g Dibutylglykolformal (78% d. Th.) mit einem Siedepunkt von 95 °C/0,1 mbar erhalten.

2232 g (8,9 Mol) des erhaltenen Dibutylglykolformals, 827 g (1,5 Mol) eines Anlagerungsproduktes von 7 Mol Ethylenoxid an ein Mol eines Oxoalkoholgemisches der Kettenlänge $C_{14}$–$C_{15}$ und 1,7 g konz. Schwefelsäure wurden in einem Destillationskolben unter einem Druck von 0,1 mbar solange auf 80 °C erhitzt, bis 332 g Butylglykol und Dibutylglykolformal übergegangen waren. Das zurückgebliebene Gemisch wurde mit Natriummethylat neutralisiert. Anschliessend wurden im Ölpumpenvakuum 1687 g Dibutylglykolformal abdestilliert, wobei die Sumpftemperatur bis 200 °C gesteigert wurde. Der verbliebene Rückstand wurde filtriert. Es wurden 1037 g Polyglykolethermischformal mit der OH-Zahl 2,0 und dem Trübungspunkt 16 °C erhalten.

Beispiele 15–22

Die Umsetzungen wurden in Analogie zu Beispiel 14 durchgeführt. Als Polyalkylenglykolether-Ausgangsmaterial wurden Ethylenoxid- und Ethylenoxid-/Propylenoxidanlagerungsorodukte von Fettalkoholgemischen der Kettenlänge $C_{12}$–$C_{18}$ (Kokosfettalkoholschnitt) und der Kettenlänge $C_{14}$–$C_{20}$ (Talgalkohol), Decanol und Nonylphenol eingesetzt. Diese Polyalkylenglykolether sind in der nachfolgenden Tabelle II durch die Alkohol- oder Phenolkomponente und die Anzahl der angelagerten Alkylenoxideinheiten charakterisiert. Bei den Ethylenoxid-/Propylenoxidanlagerungsprodukten ist die Reihenfolge der Anlagerung aus der Angabe ersichtlich; + 8 EO + 1 PO bedeutet, dass zuerst Ethylenoxid, dann Propylenoxid addiert wurde.

Tabelle II

| Beispiel | Polyglykolether | Diformal | OH-Zahl | Trübungspunkt °C |
|---|---|---|---|---|
| 15 | $C_{12-18}$–Fettalkohol + 2 EO | $(C_2H_5OC_2H_4O)_2CH_2$ | 0 | <0 |
| 16 | $C_{12-18}$–Fettalkohol + 5 EO | $(C_2H_5OC_2H_4O)_2CH_2$ | 5,3 | 36–40 |
| 17 | $C_{12-18}$–Fettalkohol + 7 EO | $(C_2H_5OC_2H_4O)_2CH_2$ | 2,5 | 42 |
| 18 | $C_{12-18}$–Fettalkohol + 7 EO | $(C_4H_9OC_2H_4O)_2CH_2$ | 0,8 | 17 |
| 19 | $C_{12-18}$–Fettalkohol + 9 EO | $(C_4H_9OC_2H_4O)_2CH_2$ | 1,6 | 29 |
| 20 | $C_{14-20}$–Fettalkohol + 14 EO | $(C_4H_9OC_2H_4O)_2CH_2$ | 1,4 | 38 |
| 21 | Nonylphenol + 9,5 EO | $(C_4H_9OC_2H_4O)_2CH_2$ | 1,0 | 7,5 |
| 22 | $C_{12-18}$–Fettalkohol + 8 EO + 1 PO | $(CH_3OCH_2CH(CH_3)O)_2CH_2$ | 1,9 | 37 |
| 23 | Decanol + 3 EO | $(CH_3O(C_2H_4O)_2)_2CH_2$ | 1,0 | 35 |

Beispiel 24

Das in Beispiel 13 beschriebene Polyalkylenglykolethergemisch wurde analog Beispiel 14 mit Dibutylglykolformal umgesetzt. Es wurde ein Mischformal mit der OH-Zahl 3,7 erhalten.

Beispiel 25

Zur Prüfung auf ihre Beständigkeit gegenüber Alkali wurden die in der folgenden Tabelle III mit A1 bis A4 bezeichneten, nach dem erfindungsgemässen Verfahren hergestellten Polyglykolethermischformalen herangezogen. Als Vergleichssubstanzen dienten die Produkte B1 bis B4, die jeweils ausgehend von demselben Polyglykolether nach den Verfahren der DE-OS 2 523 588 durch Umsetzung mit Formaldehyd und dem entsprechenden Alkohol erhalten worden waren.

Die Substanzen A1 bis A4 und B1 bis B4 wurden 3 Tage lang bei 90 °C über Ätznatron aufbewahrt. Die danach beobachtete Farbe der Proben ist in der letzten Spalte der Tabelle III verzeichnet.

Tabelle III

| Nr. | | OH-Zahl | Trübungspunkt °C | Farbe des Produktes |
|---|---|---|---|---|
| A 1 | $C_{12}H_{25}(EO)_{10}OCH_2OC_4H_9$ | 2 | 20 | wasserhell |
| B 1 | $C_{12}H_{25}(EO)_{10}OCH_2OC_4H_9$ | 19 | 31 | tiefbraun |
| A 2 | $C_{12}H_{25}(EO)_{15}OCH_2OC_4H_9$ | 0 | 37 | wasserhell |
| B 2 | $C_{12}H_{25}(EO)_{15}OCH_2OC_4H_9$ | 17 | 50 | tiefbraun |
| A 3 | $C_{12-18}H_{25-37}(EO)_9OCH_2OC_4H_9$ | 0 | 24 | wasserhell |
| B 3 | $C_{12-18}H_{25-37}(EO)_9OCH_2OC_4H_9$ | 16 | 33 | tiefbraun |
| A 4 | $C_{12}H_{25}(EO)_{10}OCH_2OC_2H_4OC_4H_9$ | 1 | 24 | wasserhell |
| B 4 | $C_{12}H_{25}(EO)_{10}OCH_2OC_2H_4OC_4H_9$ | 26 | 33 | tiefbraun |

**Patentansprüche**

1. Verfahren zur Herstellung Polyglykolethermischformalen der Formeln I oder II,

$$\begin{array}{l} R^1\text{–}CH\text{–}O\text{–}(A\text{–}O)_m\text{–}CH_2\text{–}(O\text{–}B)_q\text{–}O\text{–}R^4 \\ \quad\ | \\ R^2\text{–}CH\text{–}O\text{–}(A\text{–}O)_n\text{–}CH_2\text{–}(O\text{–}B)_q\text{–}O\text{–}R^4 \end{array} \qquad I$$

$$R^3\text{–}O\text{–}(A\text{–}O)_p\text{–}CH_2\text{–}(O\text{–}B)_q\text{–}O\text{–}R^4 \qquad II$$

in denen
$R^1$ für einen geradkettigen oder verzweigten Alkylrest mit 1 bis 18 Kohlenstoffatomen und
$R^2$ für Wasserstoff oder einen geradkettigen oder verzweigten Alkylrest mit 1 bis 17 Kohlenstoffatomen steht,
wobei die Summe der Kohlenstoffatome in $R^1$ und $R^2$ 6 bis 18 beträgt,
$R^3$ einen Mono-, Di- oder Trialkylphenylrest mit 14 bis 26 Kohlenstoffatomen, einen geradkettigen oder verzweigten Alkylrest mit 8 bis 22 Kohlenstoffatomen oder einen Rest der Formel III bedeutet,

$$\begin{array}{l} R^1\text{–}CH\text{–}O\text{–}R^5 \\ \quad\ | \\ R^2\text{–}CH\text{–} \end{array} \qquad III$$

in der $R^1$ und $R^2$ die oben angegebene Bedeutung haben und
$R^5$ einen geradkettigen oder verzweigten Alkylrest mit 1 bis 5 Kohlenstoffatomen, einen Aryl- oder Alkylarylrest mit 6 bis 9 Kohlenstoffatomen darstellt, während
$R^4$ einen geradkettigen oder verzweigten Alkylrest mit 1 bis 5 Kohlenstoffatomen,
A und B Ethylen- oder Isopropylenreste,
m und n Zahlen von 0 bis 50, p eine Zahl von 2 bis 50 und q eine Zahl von 0 bis 3 bedeuten, wobei die Summe von m + n 2 bis 50 beträgt,
dadurch gekennzeichnet, dass man
Polyglykolether der Formel IV oder V,

$$\begin{array}{l} R^1\text{–}CH\text{–}O\text{–}(A\text{–}O)_m\text{–}H \\ \quad\ | \\ R^2\text{–}CH\text{–}O\text{–}(A\text{–}O)_n\text{–}H \end{array} \qquad IV$$

$$R^3\text{–}O\text{–}(A\text{–}O)_p\text{–}H \qquad V$$

in denen $R^1$, $R^2$, $R^3$, A, m, n und p die oben angegebene Bedeutung haben,
mit einem Diformal der Formel VI,

$$R^4\text{–}O\text{–}(B\text{–}O)_q\text{–}CH_2\text{–}(O\text{–}B)_q\text{–}O\text{–}R^4 \qquad VI$$

in der $R^4$, B und q die oben angegebene Bedeutung haben,
bei 60 bis 150 °C in Gegenwart einer starken Säure umsetzt, wobei pro Mol in IV oder V vorhandener Hydroxylgruppen 2 bis 10 Mol Diformal eingesetzt werden,
den bei der Reaktion entstandenen Alkohol $R^4$–O–(B–O)$_q$H, gegebenenfalls zusammen mit Diformal, aus dem Reaktionsgemisch abdestilliert, die im Rückstand vorhandene Säure neutralisiert oder gegebenenfalls durch Filtration entfernt, das noch vorhandene Diformal abdestilliert und gegebenenfalls die anwesenden Salze durch Filtration entfernt.

2. Verfahren nach Anspruch 1, dadurch gekennzeichnet, dass die Umsetzung in Gegenwart von Phosphorsäure, p-Toluolsulfonsäure, sauren Ionenaustauschern oder sauren Zeolithen durchgeführt wird.

3. Verfahren nach Anspruch 1, dadurch gekennzeichnet, dass die Umsetzung in Gegenwart von Schwefelsäure durchgeführt wird.

4. Polyglykoläthermischformale der Formeln I und II gemäss Anspruch 1
wobei $R^3$ einen Rest der Formel III

$$\begin{array}{l} R^1\text{–}CH\text{–}O\text{–}R^5 \\ \quad\ | \\ R^2\text{–}CH\text{–} \end{array} \qquad III$$

bedeutet und alle anderen Variablen die dort angegebene Bedeutung haben.

5. Polyglykolethermischformale der Formeln I und II nach Anspruch 4, dadurch gekennzeichnet, dass $R^1$, $R^2$, $R^4$ und $R^5$ geradkettige Alkylreste bedeuten.

**Claims**

1. A process for the production of polyglycolether mixed formals corresponding to formula I or II

$$\begin{array}{l} R^1\text{–}CH\text{–}O\text{–}(A\text{–}O)_m\text{–}CH_2\text{–}(O\text{–}B)_q\text{–}O\text{–}R^4 \\ \quad\ | \\ R^2\text{–}CH\text{–}O\text{–}(A\text{–}O)_n\text{–}CH_2\text{–}(O\text{–}B)_q\text{–}O\text{–}R^4 \end{array} \qquad I$$

$$R^3\text{–}O\text{–}(A\text{–}O)_p\text{–}CH_2\text{–}(O\text{–}B)_q\text{–}O\text{–}R^4 \qquad II$$

in which

$R^1$ represents a straight-chain or branched $C_1$–$C_{18}$ alkyl group and

$R^2$ represents hydrogen or a straight-chain or branched $C_1$–$C_{17}$ alkyl group,

the sum of the carbon atoms in $R^1$ and $R^2$ amounting to between 6 and 18,

$R^3$ represents a mono-, di- or trialkyl phenyl group containing from 14 to 26 carbon atoms, a straight-chain or branched $C_8$–$C_{22}$ alkyl group or a group of formula III

$$\begin{array}{l} R^1-CH-O-R^5 \\ \quad\ \ | \\ R^2-CH- \end{array} \qquad III$$

in which

$R^1$ and $R^2$ are as defined above and

$R^5$ represents a straight-chain or branched $C_1$–$C_5$ alkyl group, a $C_6$–$C_9$ aryl or alkylaryl group whilst

$R^4$ represents a straight-chain or branched $C_1$–$C_5$ alkyl group,

A and B are ethylene or isopropylene groups,

m and n are numbers of from 0 to 50

p is a number of from 2 to 50 and

q is a number of from 0 to 3,

the sum of m + n amounting to between 2 and 50,

characterized in that

polyglycolethers corresponding to formula IV or V

$$\begin{array}{l} R^1-CH-O-(A-O)_m-H \\ \quad\ \ | \\ R^2-CH-O-(A-O)_n-H \end{array} \qquad IV$$

$$R^3-O-(A-O)_p-H \qquad V$$

in which $R^1$, $R^2$, $R^3$, A, m, n and p are as defined above, are reacted with a diformal corresponding to formula VI

$$R^4-O-(B-O)_q-CH_2-(O-B)_q-O-R^4 \qquad VI$$

in which $R^4$, B and q are as defined above,

at 60 to 150 °C in the presence of a strong acid,

from 2 to 10 moles of diformal being used per mole of hydroxyl groups present in IV or V,

the alcohol $R^4-O-(B-O)_qH$ formed during the reaction is distilled off from the reaction mixture, optionally together with diformal,

the acid present in the residue is neutralized or optionally removed by filtration,

the diformal still present is distilled off and the salts present are optionally removed by filtration.

2. A process as claimed in Claim 1, characterized in that the reaction is carried out in the presence of phosphoric acid, p-toluene sulfonic acid, acidic ion exchangers or acidic zeolites.

3. A process as claimed in Claim 1, characterized in that the reaction is carried out in the presence of sulfuric acid.

4. Polyglycolether mixed formals corresponding to formula I or II in Claim 1 in which $R^3$ is a group of formula III and all other variables are as defined there.

5. Polyglycolether mixed formals corresponding to formula I and II as claimed in Claim 4, characterized in that $R^1$, $R^2$, $R^4$ and $R^5$ are straight-chain alkyl groups.

**Revendications**

1. Procédé de préparation de formals mixtes de polyglycoléthers de formule I ou II:

$$\begin{array}{l} R^1-CH-O-(A-O)_m-CH_2-(O-B)_q-O-R^4 \\ \quad\ \ | \\ R^2-CH-O-(A-O)_n-CH_2-(O-B)_q-O-R^4 \end{array} \qquad I$$

$$R^3-O-(A-O)_p-CH_2-(O-B)_q-O-R^4 \qquad II$$

dans lesquelles $R^1$ représente un radical alkyle à chaîne droite ou ramifiée contenant 1 à 18 atomes de carbone et $R^2$ représente un atome d'hydrogène ou un radical alkyle à chaîne droite ou ramifiée contenant 1 à 17 atomes de carbone, la somme des atomes de carbone de $R^1$ et $R^2$ étant de 6 à 18, $R^3$ représente un radical mono-, di- ou trialkylphényle contenant 14 à 26 atomes de carbone, un radical alkyle à chaîne droite ou ramifiée contenant 8 à 22 atomes de carbone ou un radical de formule III:

$$\begin{array}{l} R^1-CH-O-R^5 \\ \quad\ \ | \\ R^2-CH- \end{array} \qquad III$$

dans laquelle $R^1$ et $R^2$ ont les significations indiquées ci-dessus et $R^5$ représente un radical alkyle à chaîne droite ou ramifiée contenant 1 à 5 atomes de carbone, un radical aryle ou alkylaryle contenant 6 à 9 atomes de carbone, tandis que $R^4$ représente un radical alkyle à chaîne droite ou ramifiée contenant 1 à 5 atomes de carbone, A et B représentent des radicaux éthylène ou isopropylène, m et n représentent des nombres de 0 à 50, p représente un nombre de 2 à 50 et q représente un nombre de 0 à 3, la somme de m + n étant égale à 2 à 50, caractérisé en ce qu'on fait réagir des polyglycoléthers de formule IV ou V:

$$\begin{array}{l} R^1-CH-O-(A-O)_m-H \\ \quad\ \ | \\ R^2-CH-O-(A-O)_n-H \end{array} \qquad IV$$

$$R^3-O-(A-O)_p-H \qquad V$$

dans lesquelles $R^1$, $R^2$, $R^3$, A, m, n et p ont les significations indiquées ci-dessus, avec un diformal de formule VI:

$$R^4-O-(B-O)_q-CH_2-(O-B)_q-O-R^4 \qquad VI$$

dans laquelle $R^4$, B et q ont les significations indiquées ci-dessus, à 60–150 °C en présence d'un acide fort, tout en utilisant, par mole de groupes hydroxy présents dans IV ou V, 2 à 10 moles de diformal, puis, par distillation, on sépare, hors du mélange réactionnel, l'alcool $R^4-O-(B-O)_qH$ formé lors de la réaction éventuellement avec du diformal, on neutralise l'acide présent dans le résidu ou on l'élimine éventuellement par filtration, on sépare, par distillation, le diformal encore présent et on élimine éventuellement les sels présents par filtration.

2. Procédé selon la revendication 1, caractérisé en ce qu'on effectue la réaction en présence d'acide phosphorique, d'acide p-toluène-sulfonique, d'échangeurs d'ions acides ou de zéolites acides.

3. Procédé selon la revendication 1, caractérisé en ce qu'on effectue la réaction en présence d'acide sulfurique.

4. Formals mixtes de polyglycoléthers répondant aux formules I et II selon la revendication 1, $R^3$ représentant un radical de formule III:

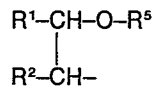

III

ainsi que toutes les autres variables qui ont la signification y indiquée.

5. Formals mixtes de polyglycoléthers répondant aux formules I et II selon la revendication 4, caractérisé en ce que $R^1$, $R^2$, $R^4$ et $R^5$ représentent des radicaux alkyle à chaîne droite.